# EUROPÄISCHE PATENTSCHRIFT

(11) **EP 1 327 110 B1**
(45) Veröffentlichungstag und Bekanntmachung des Hinweises auf die Patenterteilung: **30.11.2005**
(21) Anmeldenummer: 00966061.4
(22) Anmeldetag: 20.09.2000
(51) Int. Cl.: F24F 3/16, A61L 9/16

(54) **VORRICHTUNG ZUR REGENERATION VON RAUMLUFT**
DEVICE FOR REGENERATING AIR IN AN ENCLOSED SPACE
DISPOSITIF DE REGENERATION DE L'AIR D'ESPACES CLOS

(43) Veröffentlichungstag der Anmeldung: 16.07.2003
(73) Patentinhaber: PZ Fund Investment N.V., Curacao (AN)
(72) Erfinder: PRAHL, Helmut, 22147 Hamburg (DE); PETZ, Günter, 90439 Nürnberg (DE)
(74) Vertreter: Viering, Hans-Martin
(86) Internationale Anmeldenummer: PCT/EP2000/009210
(87) Internationale Veröffentlichungsnummer: WO 2002/025180

(56) Entgegenhaltungen:
- DE-A- 19 513 943
- DE-A- 19 842 068
- DE-U- 29 815 783
- FR-A- 2 510 891
- US-A- 5 681 533
- US-A- 5 702 507
- US-A- 5 779 769
- US-A- 5 904 896

## Beschreibung

Die Erfindung betrifft eine Vorrichtung zur Regeneration von Raumluft, insbesondere in Gebäuden.

Zur Säuberung der Raumluft von Stäuben und anderweitigen Verunreinigungen sind Partikelfilter aus unterschiedlichen Materialien, wie Geweben, Papier und Vliesstoffen oder dergleichen bekannt, während zur Vernichtung von schädlichen gas- und dampfförmigen Luftinhaltsstoffen sowie Bakterien, Schimmel oder dergleichen ionen- und ownerzeugende Baugruppen Anwendung finden, die mit dielektrisch behinderten Entladungen arbeitenden raumaufwendigen Glasröhren zum Einsatz kommen. Den bekannten Geräten ist der Nachteil gemeinsam, daß die so erzielte neutrale Luft durch Fehlen von Düften der Stimmungs- und Bewußtseinslage des Benutzers nicht gerecht wird.

US 5 904 896 offenbart ein System zur Entfernung von Luftverunreinigungen in mehreren Stufen, das einen ersten elektrostatischen Filter, anschließend einen Mikrofaserfilter, eine Adsorbereinheit für Gase und einen weiteren Partikelfilter in dieser Reihenfolge im Strömungsweg der Luft aufweist, wobei zwischen dem zweiten Filter und dem Adsorbenz ein Ozonerzeuger angeordnet ist Durch ein solches System von Verunreinigungen befreite Luft kann anschließend durch eingespritzte, Duft maskierende oder neutralisierende Stoffe oder Duftstoffe selbst angereichert werden.

Die FR 25 10 891 offenbart ein Verfahren und eine Vorrichtung zur Reinigung von Luft durch Ozon. Zur Vermeidung einer unerwünschten oder schädlichen Ozonbelastung, die in der gereinigten Luft enthalten sein kann, wird abwechselnd mit der Ozonisierung der Luft, gleichzeitig oder unabhängig davon ein Parfüm abgegeben. Die Rolle dieses Parfüms ist nicht nur die bloße Beduftung der Luft, sondern insbesondere die Zerstörung des Ozons, das in der gereinigten Luft verblieben ist.

Die Erfindung hat zur Aufgabe, eine Vorrichtung zu schaffen, die einerseits eine entkeimende und geruchsbeseitigende Wirkung auf geruchstragende Partikel, Gase oder Dämpfe, Bakterien, Keimen oder dergleichen entfaltet und andererseits eine funktionelle Beduftung der Luft ermöglicht.

Der Erfindung gemäß ist diese Aufgabe gelöst durch eine Vorrichtung mit einem Aufnahmegehäuse mit einer ersten Raumeinheit a) mit einer Vielzahl Öffnungen für Zu- und Abluft, einem motorisch getriebenen Gebläserad zur Bildung einer Durchsatzströmung, einer der Durchsatzströmung zugeordneten, nach dem Prinzip der dielektrisch behinderten Entladung arbeitenden Ionisations- und Ozonisierungsbaugruppe, einem Zuluftfilter und einer Sorptionskatalysatoreinheit als Abluftfilter für die Gebläseluft sowie einer anschließenden zweiten Raumeinheit b) mit einem Gebläse zur Bildung einer unabhängigen Luftströmung, die über Zuluft- und Abluftöffnungen ein Behältnis für die Aufnahme von Clathratan und dufttragenden Substanzen durchsetzt. Es entspricht der Erfindung, daß diese Luftströmung durch eine abgezweigte Teilluftströmung der in der Raumeinheit a) zum Einsatz kommenden Durchsatzströmung gebildet wird.

Die Vorrichtung bildet eine tragbare mobile Baueinheit, bei der durch Oxidation eine Inaktivierung der luftgetragenen Schadstoffe in einem Luftregenerationsprozess vermittels der Ionisations- und Ozonisierungsbaugruppe erfolgt, während die Clathrate und die Duftstoffe der Beseitigung von Stinkstoffen wirken bzw. der Weckung positiver Emotionen beim Benutzer auslösen. Es versteht sich, daß die Ionisations- und Ozonisierungsbaugruppe im Hinblick auf verschieden hohe Schadstoffanteile in der Raumluft zur unterschiedlich hohen Ionen- und Ozonerzeugung mit verschieden großen, bevorzugt durch Hochfrequenzgeneratoren gebildete Hochspannungen ansteuerbar sein und die Beduftung durch beliebige Duftstoffe mit unterschiedlichen Intensitäten sowie Duftcharaktäre und Duftkomponierungen erfolgen kann. Ferner wird vorgeschlagen, daß die in den Raumeinheiten a) und b) wirksamen Gebläse zu Änderungen der Luftmenge und/oder Luftgeschwindigkeit und/oder des Luftdrucks regelbar sein können.

Gemäß bevorzugter Ausgestaltung der Vorrichtung ist vorgesehen, daß die erste a) und zweite b) Raumeinheit in einem einstückig ausgebildeten Aufnahmegehäuse untergebracht sind. Auch ist möglich, die beiden Raumeinheiten a), b) in zusammengesetzten unabhängigen Gehäuseteilen auszubilden, die durch Schraub-, Steck- und/oder Klemmverbindung bzw. Klebverbindung nebeneinander funktionell zu einer Aufnahmegehäuseinheit zusammengefaßt sind.

In weiterer Ausgestaltung der Vorrichtung ist vorgesehen, daß die Ionisations- und Ozonisierungsbaugruppe, z. B. mit einem Flachbaumodul ausgestattet ist, das vorteilhaft die Ionen- und Ozonproduktion bei schwankender Grundbelastung an die Qualität der zugeführten Zuluft anpaßt, um einerseits eine sichere Zerstörung von Gasen, Dämpfen und anderweitigen Schadstoffen zu garantieren und andererseits überschüssiges Ozon weitgehend zu vermeiden. Es versteht sich, daß die Ionisations- und Ozonisierungsbaugruppe alternativ auch mit einem anderweitigen geeigneten Luftionisator, z. B. einer Siemens'schen Ionisierungsröhre betrieben werden kann.

Schließlich sieht die Vorrichtung vor, den Zuluftfilter als Partikelfilter auszulegen und beliebig, z. B. durch Textilgewebe, Vleisstoff oder dergleichen zu erstellen, während der Sorptionskatalysator als Aktivkohlefilter oder einem anderweitigen geeigneten Katalysator ausgebildet sein kann, der überschüssiges Ozon vernichtet und die Abgabe von Ozon an die Raumluft verhindert.

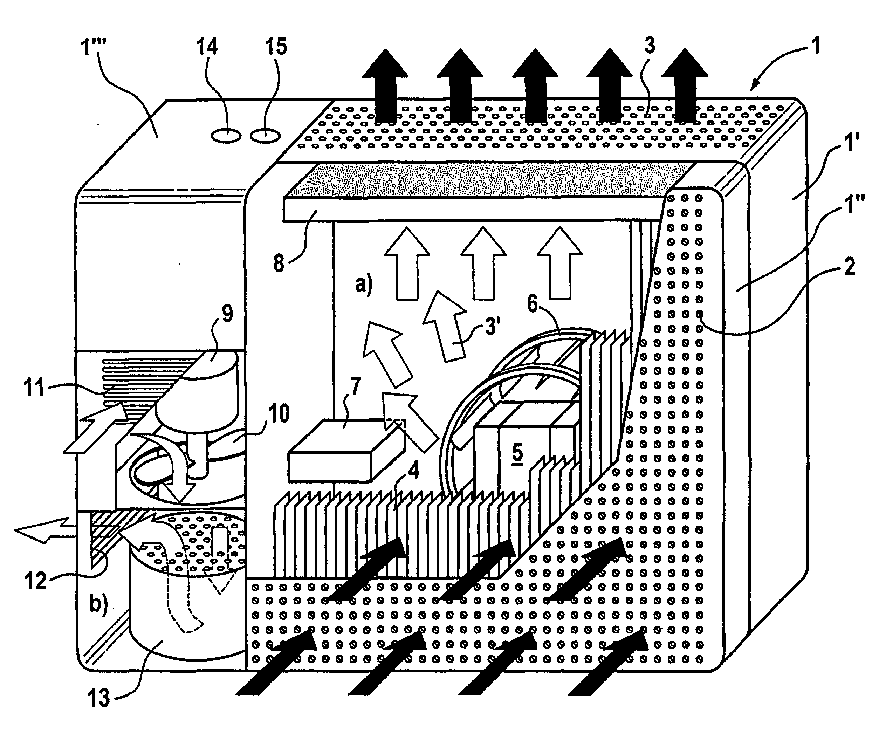

Die Erfindung ist anhand eines Ausführungsbeispiels in der Zeichnung verdeutlicht. Mit 1 ist ein Aufnahmegehäuse bezeichnet, das aus einem Rückteil 1' und einem Vorderteil 1" sowie einem Anbauteil 1"' gebildet ist, die gemeinsam eine erste Raumeinheit a) und zweite Raumeinheit b) aufweisen. Die Raumeinheit a) weist frontseitig im Vorderteil 1" eine Vielzahl als Zuluftöffnungen 2 für eine durch Pfeile 3' gekennzeichnete Durchsatzströmung auf, die über Abluftöffnungen 3 in der Oberseite des Rückteils 1' austritt. In Strömungsrichtung hinter den Zuluftöffnungen 2 ist ein Partikelfilter 4 in der Raumeinheit a) eingesetzt, der wahlweise aus Papier, Gewebe oder Vliesstoff gebildet ist. Weiter nimmt die Raumeinheit a) ein durch einen Elektromotor 5 drehbares Gebläserad 6 zur Erzeugung der Durchsatzströmung 3' und einer nach dem Prinzip der dielektrisch behinderten Entladung arbeitenden Ionisations- und Ozonisierungsbaugruppe 7, z. B. Flachbaumodul auf. Die durch die Ionisatians- und Ozonisierungsbaugruppe 7 erzeugten Ionen und das Ozon dienen der Entkeimung der Durchsatzströmung 3' von schädigenden Keimen, Bakterien, Gerüchen oder dergleichen. Das zur Entkeimung nicht benötigte überschüssige Ozon wird einem den Abluftöffnungen 3 vorgeordneten Sorptionskatalysator 8 zugeführt, dem ein Sensor zur Darstellung der Luftqualität folgen kann. Der Sorptionskatalysator 8 ist beim Ausführungsbeispiel durch einen Aktiv-Kohlefilter gebildet.

In der zweiten Raumeinheit b) ist ein durch einen Elektromotor 9 drehbarer Lüfterflügel 10 untergebracht, der über Zuluft 11 und Abluftöffnungen 12 einen Luftdurchsatz erzeugt. Desweiteren nimmt die Raumeinheit b) ein Behältnis 13 mit Clathraten und Duftstoffen auf, das vom Luftdurchsatz durchsetzt wird. Als Duftstoffe können beliebig Deostoffe, Parfüms, Desinfektionsmittel oder dergleichen Anwendung finden. Mit 14 und 15 sind Betätigungsglieder der Schalter (nicht gezeigt) für die Elektromotoren 5 und 9 bezeichnet.

Mittels der Vorrichtung ist insgesamt eine Neutralstellung der Luft durch Befreiung derselben von schädigenden Partikeln und oxidative Zerstörung von gas- und dampfförmigen Schadstoffen durch Ionisation und Ozonisierung und über den Sorptionsltatalysator 8 die Unschädlichmachung etwaiger Überschüsse an Ozon erreicht, während vermittels der in der zweiten Raumeinheit b) untergebrachten Einrichtung eine Beduftung erfolgt. Die Kombination der Luftregeneration und Duftabgabe vermittels einer einzigen mobilen Vorrichtung kann beliebig vorteilhaft in gewerblichen Räumen, Hotels, Kliniken, Aufenthaltsräumen als auch in privaten Räumen eingesetzt werden.

## Patentansprüche

1. Vorrichtung zur Regeneration von Raumluft, insbesondere in Gebäuden, mit einem Aufnahmegehäuse (I) mit einer ersten Raumeinheit a) mit einer Vielzahl Öffnungen (2, 3) für Zu- und Abluft, einem zwischen diesen angeordneten, motorisch getriebenen Gebläserad (6) zur Bildung einer Durchsatzströmung (3'), einer der Durchsatzströmung zugeordneten, nach dem Prinzip der dielektrisch behinderten Entladung arbeitenden Ionisations- und Ozonisierungsbaugruppe (7), einem Zuluftfilter (4) und einer Sozptionskatslysatoreinheit (8) als Abluftfilter für die Gebläseluft, **gekennzeichnet durch** eine anschließende zweite Raumeinheit b) mit einem Behältnis (13) für die Aufnahme von Clathraten und dufttragenden Substanzen mit einem Gebläse (9, 10) zur Bildung einer unabhängigen Luftströmung aus einer abgezweigten Teilluftströmung der in der Raumeinheit a) zum Einsatz kommenden Durchsatzströmung, die über Zuluft- (11) und Abluftöffnungen (12) das Behältnis (13) durchsetzt.

2. Vorrichtung nach Anspruch 1, **dadurch gekennzeichnet, daß** die erste a) und die zweite b) Raumeinheit in einem einstückig ausgebildeten Aufnahmegehäuse untergebracht sind.

3. Vorrichtung nach Anspruch 1, **dadurch gekennzeichnet, daß** die erste a) und die zweite b) Raumeinheit in unabhängigen Gehäuseteilen ausgebildet sind, die durch Schraub-, Steck- und/oder Klemmverbindung bzw. Klebverbindung nebeneinander funktionell zu einer Aufnahmegehäuseeinheit zusammengefaßt sind.

4. Vorrichtung nach Anspruch 1, **dadurch gekennzeichnet, daß** die Ionisations- und Ozonisierungsbaugruppe (7) mit einem Flachbaumodell ausgestattet ist.

5. Vorrichtung nach Anspruch 1, **dadurch gekennzeichnet, daß** die lonisations- und Ozonisierungsbaugruppe (7) eine Siemens'sche Ionisierungsröhre aufweist.

6. Vorrichtung nach Anspruch 1, **dadurch gekennzeichnet, daß** der Zuluftfilter (4) als Partikelfilter ausgelegt ist.

7. Vorrichtung nach Anspruch 1, **dadurch gekennzeichnet, daß** der Sorptionskatalysator (8) durch einen Aktivkohlefilter oder einen anderweitigen geeigneten Katalysator gebildet ist.

8. Vorrichtung nach Anspruch 1 und 7, **dadurch gekennzeichnet, daß** dem Sorpdonskatalysator (8) austrittsseitig ein Sensor zur quantitativen Überwachung von Ozon in der Abluft zugeordnet ist.

9. Vorrichtung nach Anspruch 1, **dadurch gekennzeichnet, daß** die in den Raumeinheiten a) und b) wirksamen Gebläse zu Änderungen der Luftmenge und/oder Luftgeschwindigkeit und/oder des Luftdrucks regelbar sind.

## Claims

1. A device for regeneration of room air, especially in buildings, comprising a receiving housing (1) with a first room unit a) with a plurality of openings (2, 3) for supply air and exhaust air, a motor-driven impeller (6) arranged therebetween to form a throughput flow (3'), an ionizing and ozonizing component (7) assigned to the throughput flow that functions according to the principle of the dielectrically impeded discharge, a supply air filter (4) and a sorption catalyst unit (8) as exhaust air filter for the blower air, **characterized by** a connected second room unit b) with a container (13) for holding clathrates and scent-bearing substances and having a blower (9, 10) to form an independent air flow from a branched partial air flow of the throughput flow used in the room unit a), which intersperses the container (13) through supply air openings (11) and exhaust air openings (12).

2. A device according to claim 1, **characterized in that** the first a) and second b) room unit are accommodated in a single-piece formed receiving housing.

3. A device according to claim 1, **characterized in that** the first a) and second b) room units are formed in adjacent independent housing parts that are functionally combined to a single receiving housing unit by means of a screw, plug-in and/or clamped connection and a glued connection, respectively.

4. A device according to claim 1, **characterized in that** the ionization and ozonizing component (7) is equipped with a flat assembly module.

5. A device according to claim 1, **characterized in that** the ionization and ozonizing component (7) has a Siemens ionization tube.

6. A device according to claim 1, **characterized in that** the supply air filter (4) is designed as a particle filter.

7. A device according to claim 1, **characterized in that** the sorption catalyst (8) is formed by an active charcoal filter or another suitable catalyst.

8. A device according to claim 1 and 7, **characterized in that** a sensor for quantitative monitoring of ozone in the exhaust air is assigned to the sorption catalyst (8) on the exit side.

9. A device according to claim 1, **characterized in that** the blowers active in the room units a) and b) are controllable for changes of the air amount and/or the air speed and/or the air pressure.

## Revendications

1. Dispositif de régénération de l'air ambiant, notamment dans des édifices, comprenant un boîtier d'admission (1) avec une première unité de volume a) avec une pluralité d'ouvertures (2, 3) pour l'air frais et l'air vicié, une roue de soufflage (6) montée entre ces dernières et entraînée par un moteur afin de créer un débit d'écoulement (3'), un sous-groupe d'ionisation et d'ozonisation (7) destiné au débit d'écoulement et fonctionnant selon le principe d'une décharge restreinte diélectrique, un filtre pour air frais (4) et une unité de catalyseur à sorption (8) servant de filtre à air vicié pour l'air soufflé, **caractérisé par** une deuxième unité de volume adjacente b) qui comprend un récipient (13) pour recevoir les clathrates et les substances porteuses d'odeurs, ainsi qu'une soufflante (9, 10) permettant de former un écoulement d'air indépendant, à partir d'un écoulement d'air partiel dérivé issu du débit d'écoulement créé dans l'unité de volume a), qui communique avec le récipient (13) par le biais d'ouvertures pour air frais (11) et pour air vicié (12).

2. Dispositif selon la revendication 1, **caractérisé en ce que** les première a) et seconde b) unités de volume sont implantées dans un boîtier d'admission fait d'une seule pièce.

3. Dispositif selon la revendication 1, **caractérisé en ce que** les première a) et seconde b) unités de volume sont formées dans des parties boîtiers indépendantes qui sont connectées toutes deux et l'une à côté de l'autre de manière fonctionnelle à une unité de boîtier d'admission par une fixation à vis, à fiches et/ou à mâchoire ou par collage.

4. Dispositif selon la revendication 1, **caractérisé en ce que** le sous-groupe d'ionisation et d'ozonisation (7) se présente sous forme d'un élément structurel plan.

5. Dispositif selon la revendication 1, **caractérisé en ce que** le sous-groupe d'ionisation et d'ozonisation (7) comporte un tube d'ionisation de Siemens.

6. Dispositif selon la revendication 1, **caractérisé en ce que** le filtre pour air frais (4) consiste en un filtre à particules.

7. Dispositif selon la revendication 1, **caractérisé en ce que** le catalyseur à sorption (8) est fabriqué à partir d'un filtre à carbone actif ou d'un autre catalyseur approprié.

8. Dispositif selon les revendications 1 et 7, **caractérisé en ce qu'**un capteur servant à contrôler la quantité d'ozone dans l'air vicié est monté sur le catalyseur à sorption (8) au niveau de sa sortie.

9. Dispositif selon la revendication 1, **caractérisé en ce que** les soufflantes opérant dans les unités de volume a) et b) peuvent être ajustées de manière à modifier la quantité d'air et/ou la vitesse de l'air et/ou la pression de l'air.
